# EUROPÄISCHE PATENTANMELDUNG

(11) **EP 2 510 879 A1**
(43) Veröffentlichungstag der Anmeldung: **17.10.2012**
(21) Anmeldenummer: 11003091.3
(22) Anmeldetag: 12.04.2011
(51) Int. Cl.: A61B 6/06, A61B 6/00

(54) **Vorrichtung zum Unterbrechen von Röntgenstrahlen**

(71) Anmelder: X-Alliance GmbH, 22335 Hamburg (DE)
(72) Erfinder: Dührssen, Rolf Nic, Dr., 21220 Seevetal (DE); Kaiser, Rolf, 26188 Edewecht (DE); Neumann, Volker, 22453 Hamburg (DE)
(74) Vertreter: Samson & Partner

(57) **Zusammenfassung**

Die Erfindung betrifft eine Vorrichtung zum Unterbrechen von Röntgenstrahlen. Die Vorrichtung umfasst zwei gegenüberliegende, auf einer gemeinsamen Achse gelagerte für Röntgenstrahlen undurchlässige Scheiben, die jeweils wenigstens einen für Röntgenstrahlen durchlässigen Bereich aufweisen. Die Vorrichtung umfasst ferner eine Antriebseinheit zum rotierenden Antreiben der zwei Scheiben, die derart ausgestaltet ist, dass sie die zwei Scheiben unabhängig voneinander antreibt. Die Erfindung betrifft auch ein Verfahren zum Unterbrechen von Röntgenstrahlen.

## Beschreibung

### GEBIET DER ERFINDUNG

Die Erfindung betrifft eine Vorrichtung und ein Verfahren zur Unterbrechung von Röntgenstrahlen.

### HINTERGRUND DER ERFINDUNG

In der Röntgentechnik ist es bekannt, ein zu untersuchendes Objekt einem pulsierenden Röntgenstrahl auszusetzen, um die Röntgenstrahldosis für den Patienten zu reduzieren. Der gepulste Röntgenstrahl wird dabei mittels einer Röntgenröhre in Kombination mit einem Generator erzeugt. Um gepulste Röntgenstrahlen zu erzeugen, wird die Hochspannung entsprechend gesteuert. Ein weiterer Vorteil gepulster Röntgenstrahlen ist ein verbesserter Bildkontrast durch die Reduzierung von Bewegungsartefakten (d.h. Beeinträchtigungen des Röntgenbildes, die durch unerwünschte Bewegung des zu untersuchenden Objekts entstehen).

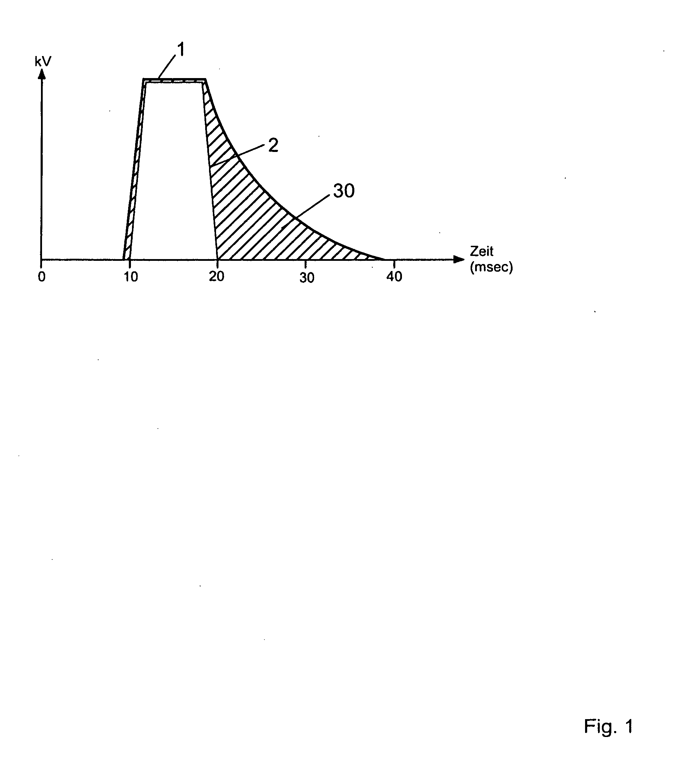

Auch zur Aufnahme von bewegten Röntgenbildern werden gepulste Röntgenstrahlen verwendet. Dazu werden typischerweise Pulsfrequenzen im Bereich 4 - 30 F/s (Bilder=Frames/Sekunde) erzeugt, so dass jeder Röntgenimpuls einer Bildaufnahme entspricht. Zur Erzeugung gepulster Röntgenstrahlen gibt es zahlreiche auf dem Markt befindliche Möglichkeiten, wie beispielsweise Grid Switch oder Grid Controlled Fluoroscopy (gittergesteuerte Fluoroskopie; Durchleuchtungsuntersuchungen mit oder ohne Kontrastmittel, das eine Verringerung der Gesamtstrahlenbelastung mit bis 80% im Vergleich zu früheren Techniken erlaubt). Hierbei werden durch spezielle Hochspannungsentladetechniken (HV-Discharge-Techniken) möglichst steile Flanken am Anfang und am Ende der Pulse erzeugt, wobei es sich nicht vermeiden lässt, dass die Flanken, insbesondere die Ausschaltflanken dennoch eine gewisse Länge aufweisen. Die Flanken (siehe Fig. 1) entsprechen einer weichen Strahlung, die vom Körper stark absorbiert wird und für den Patienten eine hohe Strahlenbelastung zur Folge hat. Um diesen Effekt zu verringern, wurde bereits vorgeschlagen, ein Kurzschließen der Hochspannungskabel durchzuführen, wofür jedoch ein hoher technischer Aufwand notwendig ist.

Eine weitere Möglichkeit, diesem Problem zu begegnen, ist aus dem Stand der Technik bekannt. Dabei wird ein gepulster Röntgenstrahl mittels einer rotierenden Unterbrechungsscheibe (Shutterscheibe), d.h. auf mechanische Weise, erzeugt. Bei diesem Verfahren wird eine rotierende Scheibe mit Öffnungen in den Strahlengang gebracht, die den Röntgenstrahl periodisch unterbricht. In diesem Zusammenhang zeigt die GB 2 132 460 A in Figur 4 eine zusammengesetzte Scheibe, die aus zwei überlagerten Scheiben besteht, wobei eine Scheibe an der Welle befestigt ist, die von dem Motor angetrieben wird und die andere Scheibe eine Manschette aufweist, die rotierbar auf der Welle durch einen Rückhaltering befestigt ist. Die beiden Scheiben haben jeweils Kreisausschnittsöffnungen und können zur Einstellung einer variablen Röntgenstrahlöffnung relativ zueinander verdreht werden. Ist die Röntgenstrahlöffnung eingestellt, sind die beiden Scheiben, wenn sie gemeinsam rotieren, arretiert, um eine Relativbewegung zueinander zu verhindern. Während eines Bestrahlungsvorgangs ist es daher nicht möglich, die Pulsweite des Strahls zu verändern.

### KURZFASSUNG DER ERFINDUNG

Es wird eine Vorrichtung zum Unterbrechen von Röntgenstrahlen bereitgestellt. Die Vorrichtung umfasst zwei gegenüberliegende, auf einer gemeinsamen Achse gelagerte für Röntgenstrahlen undurchlässige Scheiben, die jeweils wenigstens einen für Röntgenstrahlen durchlässigen Bereich aufweisen. Die Vorrichtung umfasst ferner eine Antriebseinheit zum rotierenden Antreiben der zwei Scheiben, die derart ausgestaltet ist, dass sie die zwei Scheiben unabhängig voneinander antreibt.

Ein weiterer Aspekt der Erfindung betrifft ein Verfahren zum Unterbrechen eines Röntgenstrahls zur Bildaufnahme, wobei eine Bildfrequenz und eine Gesamtdosis vorgegeben sind. Das Verfahren umfasst ein Rotieren von zwei Scheiben, die jeweils wenigstens einen für Röntgenstrahlen durchlässigen Bereich aufweisen, unabhängig voneinander im Wesentlichen senkrecht zum Röntgenstrahl, so dass Röntgenpulse entstehen. Die Pulsdauern werden geregelt aufgrund der gemessenen Dosisleistung anhand einer Phasenverschiebung der zwei Scheiben zueinander, so dass die vorgegebene Bildfrequenz und Gesamtdosis eingehalten werden.

Weitere Merkmale sind der offenbarten Vorrichtung inhärent oder sind für den Fachmann aus der folgenden detaillierten Beschreibung von Ausführungsformen und den beiliegenden Zeichnungen offensichtlich.

### KURZE BESCHREIBUNG DER ZEICHNUNGEN

Ausführungsformen der Erfindung werden nun beispielhaft und unter Bezugnahme auf die beiliegenden Zeichnungen beschrieben, in denen:
Fig. 1 den Spannungsverlauf beim Ausschalten einer gepulsten Röntgenröhre gemäß dem Stand der Technik zeigt;
Fig. 2a einen Aufbau einer Anordnung zur Aufnahme bewegter Bilder zeigt;
Fig. 2b die Funktionsweise zweier Regelkreise zur Regelung der Dosisleistung veranschaulicht;
Fig. 3 eine Unterbrechungseinheit gemäß einigen Ausführungsformen der Erfindung zeigt;
Fig. 4a eine Unterbrechungsscheibe gemäß einer Ausführungsform der Erfindung zeigt;
Fig. 4b eine weitere Unterbrechungsscheibe gemäß einer weiteren Ausführungsform der Erfindung zeigt;
Fig. 4c eine Haltescheibe gemäß einer Ausführungsform der Erfindung zeigt; und
Fig. 5a-5f die Phasenverschiebung der beiden Scheiben und den sich ergebenden Dosisverlauf veranschaulichen.

Die Zeichnungen und die Beschreibung der Zeichnungen sind von Ausführungsformen der Erfindung und nicht von der Erfindung selbst.

### BESCHREIBUNG BEVORZUGTER AUSFÜHRUNGSFORMEN

Fig. 1 zeigt einen typischen Verlauf einer Aufnahme nach Entladung, bei der die Röntgenquelle gemäß einem aus dem Stand der Technik bekannten Verfahren mittels Abschaltung gepulst wird. Bevor jedoch auf die Fig. 1 genauer eingegangen wird, sollen einige Punkte der Erfindung diskutiert werden.

Einige Ausführungsformen der Erfindung beziehen sich auf eine Vorrichtung zum Unterbrechen von Röntgenstrahlen. Die Vorrichtung umfasst zwei gegenüberliegende, auf einer gemeinsamen Achse gelagerte für Röntgenstrahlen undurchlässige Scheiben, die jeweils wenigstens einen für Röntgenstrahlen durchlässigen Bereich aufweisen. Die Vorrichtung umfasst ferner eine Antriebseinheit zum rotierenden Antreiben der zwei Scheiben, die derart ausgestaltet ist, dass sie die zwei Scheiben unabhängig voneinander antreibt.

Dies bedeutet insbesondere, dass die beiden Scheiben mit gleicher Drehzahl aber mit unterschiedlicher Phasenverschiebung der Scheiben zueinander und in entgegen gesetzter Richtung angetrieben werden können, wobei die Drehzahl der Bildfrequenz entspricht. Mit Hilfe der Vorrichtung (2 x 120° Materialausschnitt) können nun bei einer bestimmten Bildfrequenz unterschiedliche Bestrahlungszeiten (Pulszeiten, Pulsdauern) erreicht werden, was eine größere Flexibilität erlaubt. Beispielsweise kann bei einer festen Bildfrequenz von 4 F/s eine Pulsdauer zwischen 5 und 160 ms erreicht werden. Bei 10 F/s kann eine Pulsdauer zwischen 5 und 66 ms erreicht werden und bei 20 F/s eine Pulsdauer von 5 bis 33 ms. Außerdem lassen sich die Pulsdauern durch die Steuerung während des Bestrahlungsvorgangs anpassen. Andererseits können mit Hilfe der Vorrichtung bei einer fest vorgegebenen Pulsdauer, unterschiedliche Bildfrequenzen erzielt werden. Die Drehzahlen der Scheiben werden über die Wahl der gewünschten Frames pro Sekunde hin gesteuert. Bei einer Scheibe mit zwei Öffnungen halbiert sich die Drehzahl, ausgedrückt in Umdrehungen pro Sekunde, für die gewünschte Anzahl der Frames pro Sekunde. Das heißt bei 20 F/s ergibt sich eine Drehzahl von 10 Umdrehungen pro Sekunde, entsprechend 600 Umdrehungen pro Minute. Die Anzahl der Frames pro Sekunde wird wiederum durch die Anwendung, beispielsweise durch das zu untersuchende Organ, vorgegeben. Die Pulsdauer wird über eine Regelung im Hinblick auf die eingestellte Dosis geregelt. Dabei wird die Pulsdauer durch den relativen Versatz (Phasenverschiebung) der beiden Scheiben zueinander eingestellt. Da sich die Dosis mit Hilfe der Pulsdauer jedoch nur in einem eingeschränkten Bereich regeln lässt, und zwar nach oben bzw. unten durch den maximalen bzw. minimalen Öffnungsbereich (abhängig von der Geometrie der Öffnungsbereiche der beiden Scheiben) der sich gegenüberliegenden Scheibenöffnungen beschränkt, ist ein zweiter Regelkreis vorgesehen, die sich über die kV-Regelung der Röntgenröhre beeinflussen lässt. Je höher die an der Röntgenröhre angelegte Spannung, desto hochenergetischer ist die entstehende Röntgenstrahlung und damit auch die Dosis für den Patienten. Gleiches gilt bei Reduzierung des kV-Wertes, wodurch eine niedrigere Dosis appliziert wird.

Eine "für Röntgenstrahlen undurchlässige Scheibe" definiert eine Scheibe, die keine oder fast keine Röntgenstrahlen durchlässt.

Ein "für Röntgenstrahlen durchlässiger Bereich" auf der Scheibe ist in einigen Ausführungsformen der Erfindung eine Öffnung, durch die die Röntgenstrahlung dringt, während sie in anderen Ausführungsformen ein Bereich ist, der eine röntgenstrahldurchlässige Beschichtung aufweist oder der nicht mit einer röntgenstrahlundurchlässigen Beschichtung beschichtet ist.

In einigen Ausführungsformen sind die zwei Scheiben parallel zu einem feststehenden, für Röntgenstrahlen undurchlässigen Element gelagert, das einen für Röntgenstrahlen durchlässigen Bereich aufweist. In einigen dieser Ausführungsformen ist das feststehende, für Röntgenstrahlen undurchlässige Element ein Teil (z.B. eine Wand oder zwei gegenüberliegende Wände) des Gehäuses der Vorrichtung und der für Röntgenstrahlen durchlässige Bereich ist eine Öffnung (oder zwei gegenüberliegende Öffnungen) des Gehäuses, in dem sich die Vorrichtung befindet. Ein Röntgenstrahl kann die Vorrichtung also nur zu dem Zeitpunkt durchdringen, in dem die für Röntgenstrahlen durchlässigen Bereiche der beiden rotierenden Scheiben die Öffnungen des Gehäuses überlagern.

In manchen Ausführungsformen ist die Antriebseinheit ein Antriebsmotor, der über zwei (unabhängig voneinander steuerbare) Getriebe zwei Scheiben unabhängig voneinander antreibt, während in anderen Ausführungsformen die Antriebseinheit zwei Antriebsmotoren umfasst, die jeweils zum rotierenden Antreiben je einer der zwei Scheiben vorgesehen sind, wobei ein Antriebsmotor jeweils mit einer Scheibe gekoppelt ist.

In einigen Ausführungsformen sind die Antriebsmotoren so ausgestaltet, dass sie die beiden Scheiben in Betrieb (phasenversetzt oder phasengleich) gegenläufig zueinander antreiben. Dies bewirkt, dass sich die Drehmomente der beiden Scheiben gegenseitig aufheben und kein Kreiseleffekt entsteht. Ferner geben die Scheiben den Strahlengang schneller wieder frei. Die beiden Scheiben benötigen jeweils eine Lagerung, um den von einer Steuerung vorgegebenen Gegenlauf umsetzen zu können.

Wie oben bereits angesprochen umfasst die Vorrichtung in einigen Ausführungsformen ferner eine Pulssteuerung zum Steuern der Drehzahlen der beiden Antriebsmotoren umfasst, die derart ausgestaltet ist, dass sie bei einer vorgegebenen Bildfrequenz die Pulsdauer (Pulsweite) anhand einer Phasenverschiebung zwischen den Scheiben steuert. In einigen Ausführungsformen ist eine der beiden Scheibe eine Masterscheibe, die mit einer bestimmten vorgegebenen Frequenz rotiert, und die andere Scheibe ist die Slavescheibe, deren Drehzahl sich an der Masterscheibe orientiert. Um nun die Phasenverschiebung zu verändern, um eine bestimmte Pulsdauer zu erzeugen, wird die Drehzahl der Slavescheibe für eine sehr kurze Zeitdauer erhöht oder erniedrigt.

In einigen Ausführungsformen umfasst die Vorrichtung ferner eine Systemsteuerung, welche ausgelegt ist, aufgrund einer aktuell gemessenen Dosisleistung (Ist-Wert) und einem oder mehreren vorgegebenen Dosisleistung-Soll-Werten ein Steuersignal an die Pulssteuerung bereitzustellen. (Die Dosisleistung ist der Differentialquotient der Dosis nach der Zeit und wird häufig in Nanogray/Sekunde (nGy/s) angegeben.) Das Steuersignal erhöht bzw. erniedrigt die Pulsdauern (Pulsweitenmodulation, PWM) entsprechend. Diese Regelung der Pulsweite entspricht einem ersten Regelkreis. In einigen Ausführungsformen wird die Dosisleistung mittels einer in einem Bildaufnehmer untergebrachten Photodiode bestimmt, die die Helligkeit des aufgenommenen Bildes misst, wobei die gemessene Helligkeit der Dosisleistung (Ist-Wert) entspricht, die über ein Signal der Systemsteuerung bereitgestellt wird. Die Systemsteuerung verfügt über Soll-Werte und vergleicht ständig die gemessenen Ist-Werte mit den gespeicherten Soll-Werten. Die Regelung der Pulsdauern geschieht über eine sog. ABC (Automatic Brightness Control) - Regelung.

In weiteren Ausführungsformen ist die Systemsteuerung ferner an einen Röntgengenerator einer Röntgenröhre anschließbar und, falls aufgrund der gemessenen Dosis die Pulssteuerung nicht ausreichend ist (d.h. insbesondere aufgrund der Abweichung zwischen Soll-Wert und Ist-Wert die Pulsdauern länger gemacht werden müssten als es mit Hilfe der Pulssteuerung möglich ist), ist sie ausgelegt, ein Signal an den Röntgengenerator bereitzustellen. Dieses Signal erhöht oder erniedrigt entsprechend die an der Röntgenröhre anliegende Hochspannung und beeinflusst somit die applizierte Dosis. Diese Regelung entspricht einem zweiten Regelkreis, wobei beide Regelkreise in der Systemsteuerung zusammenlaufen.

In anderen Ausführungsformen umfasst die Vorrichtung ferner eine Steuerung zum Steuern der Drehzahlen der beiden Antriebsmotoren, die derart ausgestaltet ist, dass sie die Drehzahlen so steuert, dass bei einer festen Pulsdauer die Bildfrequenz in einem vorgegebenen Bereich einstellbar ist.

In einigen Ausführungsformen werden als Antriebsmotoren Flachmotoren eingesetzt, während in anderen Ausführungsformen Motoren mit Winkelgetriebe eingesetzt werden. Die Antriebsmotoren weisen beispielsweise ein Drehzahl von 4700 min⁻¹ oder 6100 min⁻¹ auf. Ferner werden in einigen Ausführungsformen der Erfindung die Antriebsmotoren mit 24 V angetrieben, da diese Spannung im Röntgengerät zur Verfügung steht. In einigen Ausführungsformen weisen die Antriebsmotoren einen Durchmesser von ca. 90 mm bis 110 mm auf und eine Bauhöhe von 30 bis 40 mm.

In einigen Ausführungsformen der Erfindung ist das Material, aus dem die Scheiben hergestellt sind, vollständig röntgenstrahlabsorbierend, um den Röntgenstrahl unterbrechen zu können. In einigen Ausführungsformen sind die Scheiben aus Wolfram hergestellt. Eine aus Wolfram bestehende Scheibe hat den Vorteil, dass sie dünn ist und somit eine geringe Bauhöhe der Unterbrechungsvorrichtung ermöglicht, die wiederum für den Einbau in bestehende Röntgengeräte notwendig ist. In anderen Ausführungsformen sind die Scheiben aus einem Eisen-Wolfram-Sinterwerkstoff hergestellt, welcher durch die Kombination der Werkstoffe Eisen und Wolfram eine durch den Wolframanteil gute absorbierende Eigenschaft und zugleich durch den Eisenanteil eine hohe Festigkeit hat. Der Eisen-Wolfram-Sinterwerkstoff weist durch den geringen Eisenanteil keine relevante Veränderung der Absorption von Röntgenstrahlung auf. In wiederum anderen Ausführungsformen sind die Scheiben aus Blei hergestellt.

In einigen Ausführungsformen sind die Scheiben mittels Laserstrahlschneiden hergestellt, während sie in anderen Ausführungsformen mittels Elektronenstrahlschneiden, Fräsen oder Wasserstrahlschneiden hergestellt werden. In wiederum anderen Ausführungsformen der Erfindung werden die Scheiben bereits in der gewünschten Form gesintert. Beim Sintern werden zumeist körnige oder pulvrige Stoffe vermischt und dann durch Erwärmung miteinander verbunden. Im Gegensatz zur reinen Schmelze werden hierbei jedoch keine oder zumindest nicht alle Ausgangsstoffe aufgeschmolzen.

In manchen Ausführungsformen ist jede Scheibe mittels eines Rillenkugellager aus Kunststoff gelagert. Ein Kunststoffkugellager hat hierbei eine hohe schwingungsdämpfende Wirkung und ermöglicht einen schmiermittelfreien Lauf der Scheiben.

In einigen Ausführungsformen weisen die beiden Scheiben jeweils zwei Öffnungen in Form eines Kreisausschnitts mit einem Mittelpunktswinkel von 120° auf. Dementsprechend befinden sich zwischen den 120° Kreisausschnitten zwei 60° Kreisausschnitte aus Material. In einigen Ausführungsformen sind die Scheiben 2,5 mm dick. In anderen Ausführungsformen weisen die beiden Scheiben jeweils zwei Öffnungen in Form eines Kreisausschnitts mit einem Mittelpunktswinkel von 90° auf.

In manchen Ausführungsformen sind die Scheiben mittels einer Zahnradverbindung jeweils mit den Motoren gekoppelt, während in anderen Ausführungsformen ein Synchronriemen zum Einsatz kommt. Der Synchronriemen erlaubt eine hohe Positioniergenauigkeit, eine geringe Geräuschentwicklung und Kosteneinsparungen durch eine längere Laufzeit und niedrigen Wartungsbedarf. In einigen Ausführungsformen hat der Synchronriemen eine Breite von weniger als 2 mm. Bei einem direkten Antrieb der Scheiben mit einem Zahnrad wird an den Scheiben jeweils eine Außenverzahnung hergestellt, in die ein am Motor befestigtes Zahnrad direkt eingreift.

In einigen Ausführungsformen ist die Vorrichtung modular einsetzbar, d.h. sie kann in einer bestehenden Röntgenvorrichtung (platziert zwischen einer Röntgenröhre und einem Kollimator) nachgerüstet werden. Daraus ergeben sich Größenbeschränkungen, so dass die Vorrichtung in einigen Ausführungsformen ein Gehäuse umfasst, das im Bereich eines Verbindungsflansches eine Höhe von 21 mm und im Bereich der Außenmotoren eine Höhe von 120 mm nicht überschreitet. In einigen Ausführungsformen weisen die Scheiben eine Dicke von weniger als 4 mm auf. In einigen Ausführungsformen haben die Scheiben jeweils einen Durchmesser von 140 mm und eine Dicke von 2,5 mm.

In manchen Ausführungsformen ist die Vorrichtung als Modul ausgebildet, das für die Aus- oder Nachrüstung von Röntgengeräten geeignet ist. Die Vorrichtung umfasst hierfür ein Gehäuse, das die beiden Scheiben und die Antriebseinheit aufnimmt und im Bereich eines Verbindungsflansches zur Verbindung mit dem Röntgengerät eine Höhe von 21 mm und im Bereich des Antriebseinheit eine Höhe von 120 mm nicht überschreitet. In einigen Ausführungsformen der Erfindung wird der obere Teil des Gehäuses von einem Blech gebildet, während der untere Teil aus einem Frästeil aus Aluminium gebildet wird. In manchen Ausführungsformen weist das Gehäuse auf seiner Oberseite eine Schnittstelle zur Ankopplung an eine Röntgenröhre (Verbindungsflansch) auf. An der Unterseite des Gehäuses befindet sich ein Adapterring zur drehbaren Befestigung des Kollimators (optisches Gerät zur Erzeugung eines parallelen Strahlenverlaufs).

In einigen Ausführungsformen ist in der unteren Gehäusehälfte eine Achse für die Lagerung der Scheiben befestigt.

In manchen Ausführungsformen ist die Vorrichtung zur Unterbrechung von Röntgenstrahlen mit einer gepulsten Röntgenröhre kombiniert (Hybrid-Vorrichtung). In diesen Ausführungsformen steuert die Systemsteuerung einen Röntgengenerator so an, dass die Röntgenröhre (durch Ein- und Ausschalten bzw. Kurzschließen) eine gepulste Röntgenstrahlung aussendet, wobei die eigentliche Durchlassstrahlung weiterhin von der Vorrichtung zur Unterbrechung von Röntgenstrahlen gesteuert wird. Dadurch wird erreicht, dass die Röntgenröhre nicht unter Dauerlast steht, d.h. thermisch nicht zu stark belastet wird, was eine längere Lebenszeit der Röntgenröhre gewährleistet.

Die erfindungsgemäße Vorrichtung und das erfindungsgemäße Verfahren lassen sich in der Röntgendiagnostik einsetzen, um bewegte Bilder zu erstellen. In diesem Zusammenhang sei die Angiografie genannt, bei der zunächst ein Katheter oder eine Injektionsnadel in das Gefäßsystem (z.B. des Herzens) eingebracht wird und dann im bzw. vor den Abgang des interessierenden Blutgefäßes positioniert wird. Bevor das Kontrastmittel verabreicht wird, wird ein normales Bild (Leeraufnahme) von der untersuchten Körperregion angefertigt. Wenn dann das Kontrastmittel über Katheter bzw. Nadel in das Gefäß gespritzt wird, werden davon in schneller Abfolge Aufnahmen gemacht. Sollten diese Bilder in digitaler Form gespeichert werden, kann man von den Angiografiebildern die Leeraufnahme subtrahieren. Störende Bildelemente, die auf beiden Bildern vorhanden sind (z. B. Knochen), werden dadurch ausgeblendet, was die Auswertung der Aufnahmen erleichtert. (Digitale Subtraktionsangiografie). Bei der Angiografie wird typischerweise eine Bildrate von 20 F/s gefordert.

Kehrt man nun zu Fig. 1 zurück, so sieht man dort den Spannungsverlauf 1 bei einer gepulsten Röntgenröhre, um beispielsweise Bilder für einen Röntgenfilm aufzunehmen. Um die kurzen Belichtungsintervalle für Echtzeitbilder möglich zu machen, wird die Röntgenstrahlung durch Abschalten der Röntgenröhre unterbrochen, wozu die Spannungsversorgung des Generators für die Abschaltzeit der Röntgenröhre abgeschaltet wird. Allerdings entsteht beim Ausschalten des Generators eine lange Flanke in Form einer negativen Exponentialfunktion (schraffierte Fläche 30), da sich die an die Röntgenröhre angelegte Spannung durch die vorhandenen Leitungskapazitäten und -induktivitäten nicht verzögerungsfrei abbauen lässt. (Der Spannungsaufbau beim Einschalten geschieht hingegen sehr viel schneller, so dass die entstehende Flanke sehr viel kleiner ist.) Die schraffierte Fläche 30 entspricht einer sog. weichen, niederenergetischen Strahlung, die vom menschlichen Körper stark absorbiert wird und eine Strahlenbelastung darstellt. Für die Bildgebung ist die weiche Strahlung nicht relevant, so dass sich die Bildqualität nicht verschlechtert.

Um den beschriebenen Ausschalteffekt zu verhindern, werden in einer Kurzschlussvariante (Stand der Technik) die Abschaltspannungen des Generators durch das gegenseitige Kurzschließen der Hochspannungskabel erreicht. So lassen sich die Abschaltflanken der Amplitude minimieren. Für das Kurzschließen ist jedoch ein hoher technischer Aufwand notwendig. Über einen isolierten Hochspannungsschalter werden die beiden, von der Röntgenröhre kommenden Hochspannungsleitungen mit hoher Geschwindigkeit kurzgeschlossen, um die schädliche Weichstrahlung zu verhindern.

Mittels der erfindungsgemäßen mechanischen Vorrichtung zum Unterbrechen von Röntgenstrahlen (Spannungsverlauf 2) lassen sich diese Flankenzeiten und damit die Patienten-Dosisbelastung reduzieren.

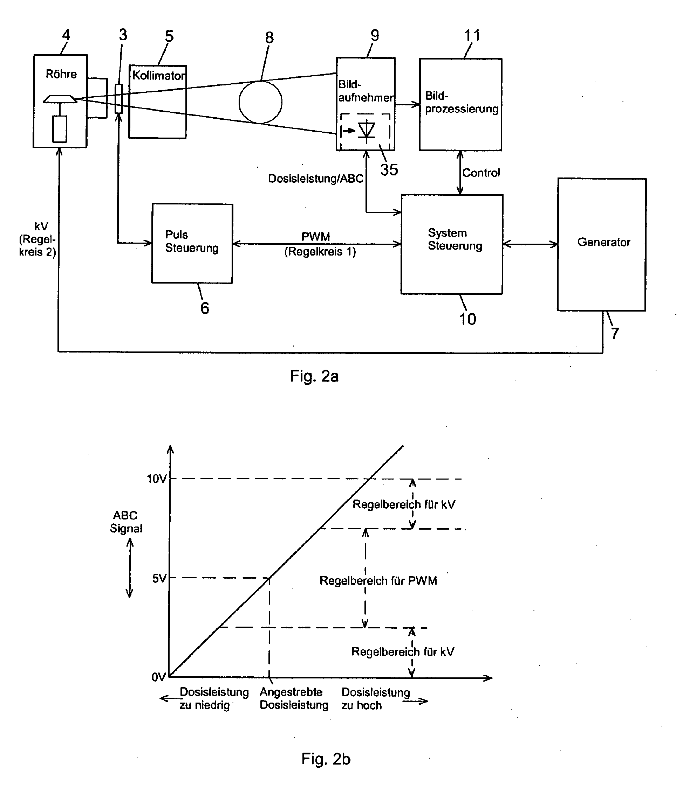

Fig. 2a zeigt eine erfindungsgemäße Unterbrechungsvorrichtung 3 (im Folgenden Shuttereinheit 3), die als nachgerüstetes Modul zwischen einer Röntgenröhre 4 und einem Kollimator 5 angeordnet ist. Der Kollimator 5 dient der Positionierung und Einblendung des Röntgenstrahls, um die Strahlenbelastung für einen Patienten zu minimieren. Die Shuttereinheit 3 wird von einer Pulssteuerung 6 gesteuert. Es wird dabei insbesondere die Drehzahl der Antriebsmotoren der beiden Unterbrechungsscheiben gesteuert. Dadurch, dass zwei Scheiben zueinander gegensinnig angetrieben werden, sind die Schaltflanken der Strahlungsunterbrechung um den Faktor zwei schneller zu realisieren. Durch die gegensinnige Drehbewegung wird wie durch eine sich öffnende und schließende Schere die Strahlung schneller beendet bzw. gestartet. D.h. gegenüber einer sich gegenüber einer ruhenden Scheibe drehenden Scheibe erreicht man durch gegenseitige Drehung beider Scheiben doppelte Verschluss- und Öffnungsgeschwindigkeit. Die Röntgenstrahlung durchdringt in kurzen Abständen (gepulster Form) ein zu untersuchendes Objekt 8 (z.B. einen Patienten) und trifft auf einen Bildaufnehmer 9, wo mit einer Bildfrequenz von 20 F/s Bilder aufgenommen werden. Im Bildaufnehmer 9 befindet sich außerdem eine Detektionseinheit 35 zur Detektion der Dosisleistung. In der vorliegenden beispielhaften Ausführungsform ist die Detektionseinheit 35 eine Photodiode, die die Helligkeit des aufgenommenen Bildes (oder eines Teils des aufgenommenen Bildes) misst. Dieses Dosisleistungssignal wird wiederum verwendet, um mittels einer relativen Phasenverschiebung der beiden Scheiben 13, 14 die Pulsweite zu regeln (1. Regelkreis). Sollte die Abweichung zwischen gemessener Dosisleistung und Soll-Dosisleistung zu groß werden, wird zusätzlich die Hochspannung eines Spannungsgenerators 7 zur Regelung der Dosis benutzt (2. Regelkreis). Es werden also zwei Regelkreise benutzt, wobei ein Regelkreis für kleine Dosisabweichungen (PWM) vorgesehen ist, der die Scheiben der Shuttereinheit 3 ansteuert, und ein Regelkreis für große Dosisabweichungen vorgesehen ist, der die kV-Regelung der Röntgenröhre 4 ansteuert. Bei 10 F/s kann das Pulssignal beispielsweise durch die entsprechende Phasenverschiebung der beiden Scheiben zueinander zwischen 0 und 25 Millisekunden gesteuert werden, in der Praxis sollte der Regelbereich im Bereich 8 bis 18 Millisekunden gewählt werden. Die zur Erzeugung der Röntgenstrahlung benötigte Spannung wird von einem Spannungsgenerator 7 erzeugt, der wiederum von einer Systemsteuerung 10 gesteuert wird. Die Pulssteuerung 6 steuert die Scheiben der Shuttereinheit 3 an und wird selbst wiederum von der Systemsteuerung 10 angesteuert. Der Bildaufnehmer 9 gibt das aufgenommene Bild an eine Bildprozessierung 11 (z.B. in Form einer Workstation) weiter. Die Bildprozessierung 11 erzeugt und verarbeitet die Bilder. Zwischen der Bildprozessierung 11 und der Systemsteuerung 10 ist eine Kommunikationsverbindung vorgesehen für die Synchronisation zur Übernahme der Bilddaten und der Bereitschaft. D.h. die Bildakquisition kann nur gestartet werden, wenn die Bildprozessierung 11 bereit ist. Über diese Verbindung sieht der Benutzer auch, dass der Röntgengenerator 7 und die Systemsteuerung 10 Aufnahmebereitschaft zeigen. Somit ist diese Verbindung wichtig zum Anzeigen der Gesamtsystembereitschaft.

Es sei darauf hingewiesen, dass die Darstellung der Fig. 2a lediglich als schematisches Funktionsbild zu verstehen ist. In einigen Ausführungsformen der Erfindung sind die Pulssteuerung 6 und die Systemsteuerung 10 Bestandteil der Shuttereinheit 3.

Fig. 2b veranschaulicht die Funktionsweise der Regelung der Dosisleistung anhand der oben bereits beschriebenen zwei Regelkreise. An der y-Achse ist die Stärke eines ABC-Signals in Volt angetragen, während die x-Achse die Dosisleistung zeigt. Ein Spannungswert von 5V entspricht einer angestrebten Dosisleistung (Soll-Wert). Liefert das ABC-Signal einen Spannungswert ungefähr in einem Intervall zwischen 2.5V und 7.5V, so kann die Dosisleistung mittels der Pulsweitenmodulation (d.h. Anpassung der Pulsdauern) geregelt werden. Ist das ABC-Signal außerhalb dieses Bereichs, d.h. zwischen 0V und 2.5V bzw. 7.5V und 10V, so reicht die Pulsweitenmodulation nicht aus, um die Dosisleistung zu regeln, so dass auf die kV-Regelung zurückgegriffen wird, um die Dosisleistung auf den gewünschten Wert von 5V zurückzubringen.

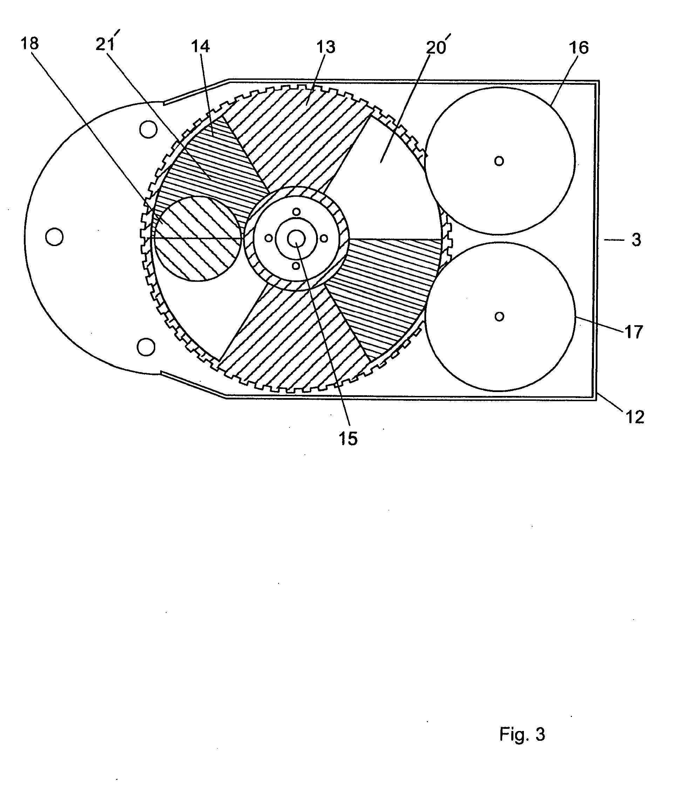

Fig. 3 zeigt den inneren Aufbau der in Fig. 2a dargestellte Shuttereinheit 3, die ein flaches Gehäuse 12 umfasst, in dem eine in das Gehäuse 12 gepresste Achse 17 vorgesehen ist. Auf der Achse 17 sind zwei gegenüberliegende Scheiben 13, 14 gelagert, die von nebeneinander angeordneten 24V-Antriebsmotoren 15, 16 angetrieben werden. Auf der Achse 17 sind zwei Kugellager (nicht gezeigt) jeweils mit ihrem Innenring über kleine Abstandshülsen durch eine Spannschraube fixiert. Der Abstand zwischen den beiden Kugellagern beträgt weniger als 1,2 mm. Jede der Scheiben 13, 14 ist durch zwei rotationssymmetrische Haltescheiben, die mit den Scheiben 13, 14 verschraubt sind, mit den Kugellagern verbunden, wobei die Haltescheiben (nicht gezeigt) sich dabei auf den Außenrand der Kugellager spannen. Der Innendurchmesser der Drehteile und der Scheiben 13, 14 sind mit Passungen versehen, um den Rundlauf zu gewährleisten. Die beiden Antriebsmotoren 15, 16 sind mittels Schrauben mit dem Gehäuse 12 verschraubt, wobei der Antriebsmotor 15 die Scheibe 13 antreibt und der Antriebsmotor 16 die Scheibe 14. Die beiden Scheiben 13, 14 sind baugleich, aus einem gesinterten Eisen-Wolfram-Werkstoff hergestellt und weisen jeweils zwei Öffnungen 20', 21' in Form von Kreisausschnitten auf. In Betrieb (d.h. bei einer Aufnahme) werden die beiden Scheiben 13, 14 von den Antriebsmotoren 16, 17 gegenläufig (phasengleich oder phasenversetzt) angetrieben. Das Gehäuse 12 weist auf der Unterseite (und auf der nicht gezeigten Oberseite) jeweils eine Öffnung 18 für die von der Röntgenröhre 4 emittierte Röntgenstrahlung auf. Wenn die beiden Scheiben 13, 14 rotieren und ihre Öffnungen 20', 21' sich über der Öffnung 18 des Gehäuses 12 treffen, durchdringt die Röntgenstrahlung für eine kurze Zeitdauer das Gehäuse 12 ungehindert. Sie durchleuchtet das zu untersuchende Objekt 8, trifft auf den Detektor 9 und eine Bildaufnahme wird gemacht. Für die Untersuchung eines bewegten Objekts sind beispielsweise 20 Bildaufnahmen pro Sekunde (20 F/s) notwendig. Durch die Verwendung von zwei Kreisausschnitten ergibt sich eine Drehgeschwindigkeit von 10 F/s. Umgerechnet auf die Minute ergeben sich 600 F/min. Trifft die Röntgenstrahlung auf den Materialteil einer der beiden Scheiben 13, 14, so wird sie absorbiert. Die beiden Antriebsmotoren 15, 16 sind mittels eines Synchronriemens mit den Scheiben 13, 14 gekoppelt, auf deren Umfang jeweils Zahnlücken 23 für den Synchronriemen angeordnet sind.

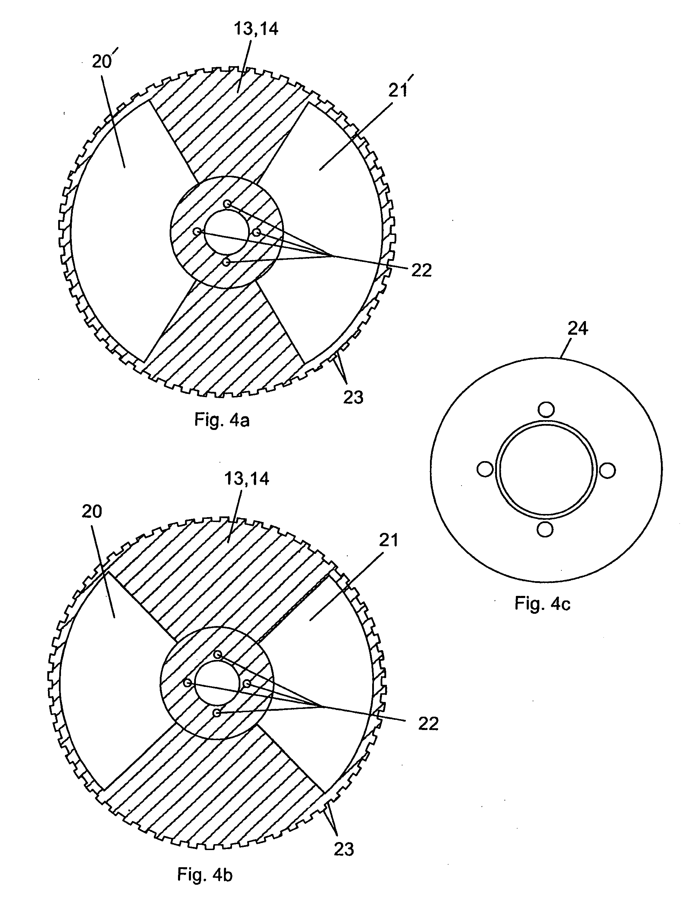

Fig. 4a zeigt den Aufbau einer Unterbrechungsscheibe 13, 14 genauer. In die Scheibe 13, 14 mit einem Außendurchmesser von 160 mm sind zwei gegenüberliegende Kreisausschnitte 20', 21' von 120° eingebracht, welche so angeordnet sind, dass sie für eine bestimmte Zeitdauer den Strahlengang für die Röntgenstrahlung freigeben. Die Scheibe 13, 14 ist 2,5 mm dick. Zwischen den 120° Kreisausschnitten 20', 21' bleibt zweimal 60° Material stehen. In der Mitte der Scheibe 13, 14 befinden sich vier Bohrungen 22 für die Befestigung der Scheibe 13, 14 mittels einer Haltescheibe 24 am Kugellager. Am Umfang der Scheibe 13, 14 sind Zahnlücken 23 für einen Synchronriemen angeordnet.

Fig. 4b zeigt den Aufbau einer weiteren Unterbrechungsscheibe 13, 14, bei der die Kreisausschnitte 20, 21 einen Mittelpunktswinkel von 90° bilden.

Fig. 4c zeigt eine rotationssymmetrische Haltescheibe 24, mittels der jede der Unterbrechungsscheiben 13, 14 mit dem Kugellager verbunden ist. Die Haltescheibe 24 spannt sich dabei auf den Außenrand des Kugellagers.

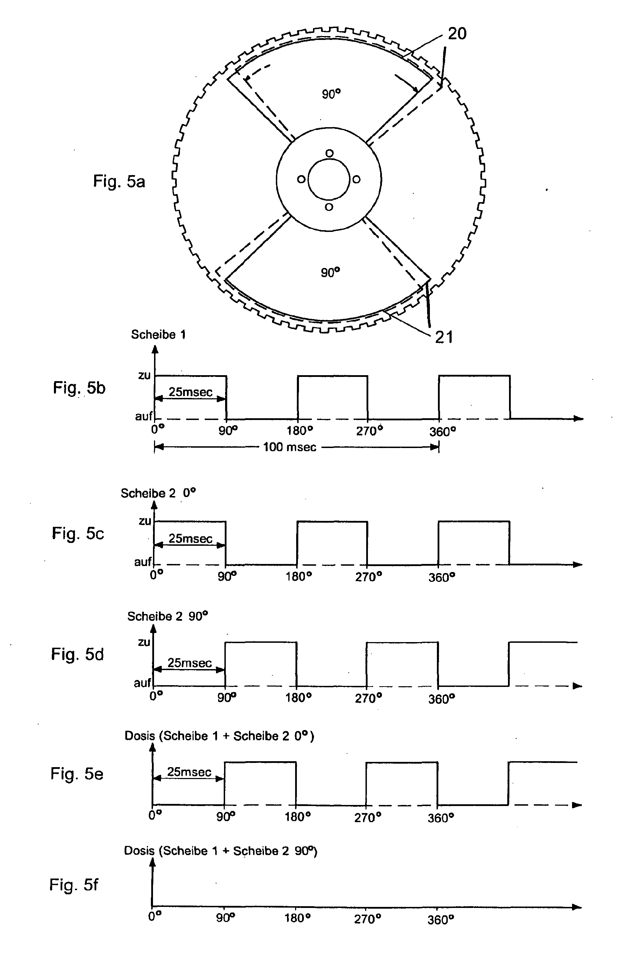

Fig. 5a zeigt eine momentane Phasenverschiebung von 5° zwischen den beiden Scheiben 13, 14, die jeweils zwei Öffnungen 20, 21 von 90° aufweisen, so dass sich zwei momentane Öffnungen von 85° bilden. Es sei darauf hingewiesen, dass in der Regel nur eine der beiden 85° Öffnungen die Öffnung 18 des Gehäuses 12 (nicht gezeigt) überlagert, so dass nur dort der Röntgenstrahl die Shuttereinheit 3 durchdringen kann.

In Fig. 5b wird der Verlauf zwischen den Zuständen "offen" und "zu" in Bezug auf eine bestimmte Stelle, z.B. die Öffnung 18 des Gehäuses 12, dargestellt. Für 25 Millisekunden ist die Öffnung 18 geschlossen, da in diesem Zeitbereich der 90°-Material-Kreisausschnitt der Scheibe 13 über die Öffnung 18 streicht. Dann überstreicht für 25 Millisekunden die Öffnung 20 der Scheibe 13 die Öffnung 18 des Gehäuses 12, dann wieder für 25 Millisekunden der geschlossene Materialteil der Scheibe 13 die Öffnung 18 und so weiter.

Fig. 5c zeigt den Verlauf zwischen den Zuständen "offen" und "zu" für eine zweite Scheibe 14, die phasengleich zur ersten Scheibe 13 rotiert.

Fig. 5d zeigt nun den Verlauf zwischen den Zuständen "offen" und "zu" für die zweite Scheibe 14, die mit einer Phasenverschiebung von 90° bezüglich der ersten Scheibe 13 (Fig. 5b) rotiert.

Fig. 5e zeigt den Dosisverlauf, wenn die Scheibe 13 aus Fig. 5b und die Scheibe 14 aus Fig. 5c phasengleich rotieren. Wie ersichtlich, ist die Dosis bei einem Drehwinkel zwischen 90° und 180° sowie 270° und 360° vorhanden, d.h. genau dann wenn die Öffnungen 20, 21 beider Scheiben 13, 14 über der Öffnung 18 des Gehäuses 12 liegen.

Fig. 5f zeigt den Dosisverlauf, wenn die erste Scheibe 13 gemäß dem in Fig. 5a dargestellten Verlauf rotiert und die zweite Scheibe 14 gemäß dem in Fig. 5d (Phasenverschiebung um 90°) dargestellten Verlauf rotieren. In diesem Fall ist die Öffnung 18 des Gehäuses 12 zu jedem Zeitpunkt von einem Materialabschnitt der beiden Scheiben 13, 14 bedeckt, so dass keine Strahlung das Gehäuse 12 durchdringt.

Die beiden Dosisverläufe in 5e und 5f zeigen zwei Extremfälle des Dosisverlaufs. Während in Fig. 5e die beiden Scheiben 13, 14 phasengleich rotieren und somit die für (die gegebenen Scheiben 13, 14 mit 90° Öffnungen 20, 21) maximale Strahlung durchlassen, rotieren die Scheiben 13, 14 in Fig. 5f derart, dass sie zu jedem Zeitpunkt jegliche Strahlung blockieren. Rotieren beide Scheiben 13, 14 nun mit einer Phasenverschiebung zwischen 0° und 90°, so ist klar, dass beliebige andere Pulsweiten zwischen 0 und 25 Millisekunden erzeugt werden können. Die Phasenverschiebungen werden mittels des Regelkreises bestimmt. Der Regelkreis ist im Verhältnis zur Aufnahmedauer so schnell, dass keine mathematische Vorhersage getroffen werden muss, welche Phasenverschiebung über die gesamte Aufnahmedauer der Bestrahlung verwendet werden sollte, um die passende Dosis zu erhalten.

Wie oben bereits angesprochen, werden durch den ersten Regelkreis die Pulsweiten (innerhalb bestimmter Grenzen) ständig anhand des Vergleichs der aktuell gemessenen Dosisleistung mit einer Soll-Wert-Dosisleistung geregelt. Ferner ist ein zweiter Regelkreis über die kV-Regelung der Röntgenröhre vorgesehen, um eine Regelung der Pulshöhen (d.h. der Pulsstärken) zu verwirklichen, falls eine Regelung des Dosisverlaufs außerhalb des mittels Phasenverschiebung der Scheiben möglichen Dosisverlaufs notwendig sein sollte.

Obgleich bestimmte Vorrichtungen hier beschrieben wurden, die gemäß den Lehren der Erfindung konstruiert sind, ist der Schutzbereich dieses Patents nicht auf diese beschränkt. Vielmehr deckt dieses Patent alle Ausführungsformen der Lehren der Erfindung ab, die entweder wörtlich oder im Sinne der Äquivalenz unter den Schutzbereich der angefügten Patentansprüche fallen.

## Patentansprüche

1. Vorrichtung zum Unterbrechen von Röntgenstrahlen, umfassend
zwei gegenüberliegende, auf einer gemeinsamen Achse (17) gelagerte für Röntgenstrahlen undurchlässige Scheiben (13, 14), die jeweils wenigstens einen für Röntgenstrahlen durchlässigen Bereich (20, 21) aufweisen, und
eine Antriebseinheit (15, 16) zum rotierenden Antreiben der zwei Scheiben (13, 14), die derart ausgestaltet ist, dass sie die zwei Scheiben (13, 14) unabhängig voneinander antreibt.

2. Vorrichtung gemäß Anspruch 1, wobei die zwei Scheiben (13, 14) parallel zu einem feststehenden, für Röntgenstrahlen undurchlässigen Element gelagert sind, das einen für Röntgenstrahlen durchlässigen Bereich aufweist.

3. Vorrichtung gemäß Anspruch 2, wobei das feststehende, für Röntgenstrahlen undurchlässige Element ein Teil des Gehäuses (12) der Vorrichtung ist und der für Röntgenstrahlen durchlässige Bereich eine Öffnung (18) des Gehäuses (12) ist.

4. Vorrichtung gemäß einem der vorhergehenden Ansprüche, wobei die Antriebseinheit (15, 16) zwei Antriebsmotoren (15, 16) zum rotierenden Antreiben jeweils einer der zwei Scheiben (13, 14) umfasst.

5. Vorrichtung gemäß einem der vorhergehenden Ansprüche, wobei die Antriebseinheit derart ausgestaltet ist, dass sie die Scheiben (13, 14) gegenläufig antreibt.

6. Vorrichtung gemäß Anspruch 4 oder 5, welche ferner eine Pulssteuerung (6) zum Steuern der Drehzahlen der zwei Antriebsmotoren (15, 16) umfasst, die derart ausgestaltet ist, dass sie bei einer vorgegebenen Bildfrequenz die Pulsdauer anhand einer Phasenverschiebung zwischen den Scheiben (13, 14) steuert.

7. Vorrichtung gemäß Anspruch 6, welche ferner eine Systemsteuerung (10) umfasst, die ausgelegt ist, aufgrund einer gemessenen Dosisleistung ein Steuersignal an die Pulssteuerung (6) bereitzustellen, die Pulsdauer entsprechend zu erhöhen oder zu erniedrigen.

8. Vorrichtung gemäß Anspruch 7, wobei die Systemsteuerung (10) ferner an einen Spannungsgenerator (7) einer Röntgenröhre (4) anschließbar ist und, falls aufgrund der gemessenen Dosisleistung die Pulssteuerung (6) nicht ausreichend ist, sie ausgelegt ist, ein Signal an den Spannungsgenerator (7) bereitzustellen, die Spannung der Röntgenröhre (4) entsprechend zu erhöhen oder zu erniedrigen.

9. Vorrichtung gemäß einem der vorhergehenden Ansprüche, wobei jede Scheibe (13, 14) mittels eines eigenen Rillenkugellagers aus Kunststoff gelagert ist.

10. Vorrichtung gemäß einem der vorhergehenden Ansprüche, wobei die für Röntgenstrahlen durchlässigen Bereiche (21, 22) Öffnungen in den Scheiben (13, 14) sind.

11. Vorrichtung gemäß einem der vorhergehenden Ansprüche, wobei die Scheiben (13, 14) aus einem Eisen-Wolfram-Sinterwerkstoff, Wolfram oder Blei hergestellt sind.

12. Vorrichtung gemäß einem der vorhergehenden Ansprüche, die als Modul ausgebildet ist, das für die Aus- oder Nachrüstung von Röntgengeräten geeignet ist, wobei das Modul eine mechanische Schnittstelle zur Ankopplung an das Röntgengerät aufweist sowie eine elektrische Schnittstelle, die einen Anschluss zu einer Detektionseinheit (35) zur Detektion der Dosisleistung und einen Anschluss zu einem Röntgengenerator (7) aufweist.

13. Röntgenvorrichtung, umfassend eine Vorrichtung zum Unterbrechen von Röntgenstrahlen gemäß einem der vorhergehenden Ansprüche, eine Röntgenröhre (4) und einen Kollimator (5), wobei die Vorrichtung zum Unterbrechen von Röntgenstrahlen zwischen der Röntgenröhre (4) und dem Kollimator (5) platziert ist.

14. Röntgenvorrichtung gemäß Anspruch 13, welche ferner einen Röntgengenerator (7) umfasst, wobei die Systemsteuerung (10) den Röntgengenerator (7) so ansteuert, dass die Röntgenröhre (4) gepulste Röntgenstrahlung aussendet.

15. Verfahren zum Unterbrechen eines Röntgenstrahls zur Bildaufnahme, wobei eine Bildfrequenz und eine Gesamtdosis vorgegeben sind, welches umfasst:
Rotieren von zwei Scheiben (13, 14), die jeweils wenigstens einen für Röntgenstrahlen durchlässigen Bereich (20, 21) aufweisen, unabhängig voneinander im Wesentlichen senkrecht zum Röntgenstrahl, so dass Röntgenpulse entstehen, und
Regeln der Pulsdauern aufgrund der gemessenen Dosisleistung anhand einer Phasenverschiebung der zwei Scheiben (13, 14) zueinander, so dass die vorgegebene Bildfrequenz und Gesamtdosis eingehalten werden.
